# EUROPÄISCHE PATENTANMELDUNG

(11) **EP 4 549 697 A1**
(43) Veröffentlichungstag der Anmeldung: **07.05.2025**
(21) Anmeldenummer: 23207762.8
(22) Anmeldetag: 03.11.2023
(51) Int. Cl.: E21C 39/00, E21D 9/00, E21D 9/12, G01N 15/00, G01N 19/00, G01N 33/24, G01N 11/10, G01N 35/00

(54) **VERFAHREN UND VORRICHTUNG ZUM BESTIMMEN EINES GEOTECHNISCHEN PARAMETERS EINES AUSBRUCHMATERIALS**

(71) Anmelder: HERRENKNECHT AKTIENGESELLSCHAFT, 77963 Schwanau (DE); Studiengesellschaft für Tunnel und Verkehrsanlagen - STUVA - e.V., 50827 Köln (DE); Technische Hochschule Köln, 50679 Köln (DE)
(72) Erfinder: Heim, Andre, 77948 Friesenheim (DE); Kassel, Andreas, 77866 Rheinau (DE); Thienert, Christian, 40699 Erkrath (DE); Gutberlet, Lara, 51065 Köln (DE); Budach, Christoph, 45277 Essen (DE); Müller, Pierre, 51145 Köln (DE)
(74) Vertreter: Klickow & Wetzel PartGmbB

(57) **Zusammenfassung**

Die Erfindung betrifft ein Verfahren zum Bestimmen wenigstens eines geotechnischen Parameters eines Ausbruchmaterials einer Tunnelbohrmaschine, wobei das Ausbruchmaterial auf eine Fördervorrichtung (2) zum Transportieren des Ausbruchmaterials aufgegeben wird, ein Anprallkörper (5) derart in einem Förderstrom (3) des Ausbruchmaterials angeordnet wird, dass er zumindest teilweise in das Ausbruchmaterial eintaucht, wobei das Ausbruchmaterial in dem Förderstrom (3) eine Strömungsgeschwindigkeit aufweist, die entweder vorgegeben ist oder gemessen wird, eine Messgröße, die einer von dem Ausbruchmaterial des Förderstroms (3) auf den Anprallkörper (5) ausgeübten Kraft entspricht, gemessen wird, wobei eine zeitliche Folge von Messwerten der Messgröße erfasst wird, und aus einem Abschnitt der Folge von Messwerten der wenigstens eine geotechnische Parameter bestimmt wird, indem der Abschnitt der zeitlichen Folge von Messwerten einer Datenverarbeitungseinrichtung eingegeben und von dieser derart verarbeitet wird, dass wenigstens ein dem wenigstens einen geotechnischen Parameter entsprechender Ausgabewert ausgegeben wird, wobei der Verarbeitung der Messwerte entweder die vorgegebene Strömungsgeschwindigkeit zugrunde liegt oder ein Wert für die gemessene Strömungsgeschwindigkeit eingegeben und bei der Verarbeitung der Messwerte berücksichtigt wird.

## Beschreibung

Die Erfindung betrifft ein Verfahren zum Bestimmen wenigstens eines geotechnischen Parameters eines Ausbruchmaterials einer Tunnelbohrmaschine.

Art und Zusammensetzung des Ausbruchmaterials von Tunnelbohrmaschinen (TBM) hängen naturgemäß von der Beschaffenheit des an der Ortsbrust anstehenden Gebirges (Fels oder Boden) ab, darüber hinaus auch von der Art des Vortriebs und der dabei dem Ausbruchmaterial zugesetzten Stoffe.

In weichen, bindigen Böden werden bevorzugt Vortriebsmaschinen mit Erddruckstützung eingesetzt, sogenannte Erddruckschild-Tunnelbohrmaschinen. Bei den Erddruckschilden dient ein Erdbrei aus Ausbruchmaterial und Wasser als plastisches Stützmedium. Ein werkzeugbestücktes, rotierendes Schneidrad der TBM wird an die Ortsbrust gedrückt und löst den anstehenden Boden. Über Öffnungen gelangt dieses Ausbruchmaterial in die Abbaukammer, wo es sich mit bereits vorhandenem Erdbrei vermischt. Eine Förderschnecke transportiert das Ausbruchmaterial vom Boden der Abbaukammer auf ein Förderband. Durch das Zusammenspiel von Förderrate der Förderschnecke und Vortriebsgeschwindigkeit kann dabei präzise der Stützdruck des Erdbreis gesteuert werden. Mittels Erddrucksensoren in der Abbaukammer werden die Druckverhältnisse kontinuierlich überwacht. Somit können alle Vortriebsparameter auch bei wechselnden geologischen Bedingungen vom Maschinenfahrer optimal aufeinander abgestimmt werden. Dies ermöglicht den nötigen Ausgleich der Druckverhältnisse an der Ortsbrust, verhindert ein unkontrolliertes Eindringen des Bodens in den unter atmosphärischem Druck stehenden Teil der Tunnelbohrmaschine und schafft so die Voraussetzung für einen schnellen und setzungsarmen Vortrieb.

Nicht alle Böden verfügen im natürlichen Zustand über ideale Eigenschaften für einen Erddruckschild-Vortrieb. Der Einsatzbereich des Verfahrens kann allerdings durch Bodenkonditionierung erweitert werden. Dabei werden plastische Verformbarkeit, Konsistenz und Wasserdurchlässigkeit der Geologie durch Zugabe von verschiedenen Konditionierungsmitteln, wie Wasser, Tone oder Schaum, verändert. Erddruckschilde können so auch in heterogenen Böden mit veränderlichen Kies-, Sand- oder Wasseranteilen oder an sich nicht standfesten Gebirgen gute Vortriebsleistungen erzielen.

Wird dem Ausbruchmaterial jedoch zu viel Wasser, Tone und/oder Schaum zugemischt, ist das beispielsweise von der Förderschnecke auf das Förderband transportierte Ausbruchmaterial aufgrund einer fließfähigen Konsistenz schlecht deponierbar; es müsste beispielsweise in Gruben oder Absetzbecken zwischengelagert oder weiterbehandelt werden. Dieses führt ggf. zu erhöhten Kosten. Darüber hinaus gibt es für die Nutzung von Deponien häufig die Vorgabe, dass eine Mindest-Scherfestigkeit des zu deponierenden Ausbruchmaterials von 10 kN/m² nicht unterschritten werden darf.

Bei solchen und weiteren Anwendungen ist es deshalb erwünscht, im laufenden Betrieb geotechnische, insbesondere rheologische Parameter des aus der Abbaukammer geförderten Ausbruchmaterials, wie beispielsweise Viskosität und Fließgrenze, die (Flügel-)Scherfestigkeit oder das Setzmaß ("slump"; gemäß DIN EN 12350-2), zu bestimmen. Beispielsweise erfordert die Bestimmung des Setzmaßes (nach DIN EN 12350-2) die Entnahme einer Probe aus dem Förderstrom und - im Rahmen eines Setzversuchs ("slump test") - das Befüllen einer kegelstumpfförmigen Metallform vorgegebener Abmessungen, ein definiertes Abheben der Metallform und das Messen der Differenz zwischen Metallformhöhe und der Höhe des nach dem Abheben zusammengesunkenen Ausbruchmaterial-Kegelstumpfes. Dies beansprucht Zeit und ist nicht vollständig automatisierbar; insbesondere ist keine kontinuierliche Messwerterfassung möglich. Ähnliches gilt für die Bestimmung alternativer geotechnischer Parameter, wie dem Ausbreitmaß, der Flügelscherfestigkeit oder der dynamischen Viskosität und der Fließgrenze.

Aufgabe der Erfindung ist es daher, im laufenden Betrieb automatisiert geotechnische Parameter des aus der Abbaukammer geförderten Ausbruchmaterials zu bestimmen.

Diese Aufgabe wird erfindungsgemäß durch ein Verfahren zum Bestimmen wenigstens eines geotechnischen Parameters eines Ausbruchmaterials einer Tunnelbohrmaschine mit den Merkmalen des Anspruchs 1 bzw. durch eine Vorrichtung zum Bestimmen wenigstens eines geotechnischen Parameters eines in einer Tunnelbohrmaschine anfallenden Ausbruchmaterials mit den Merkmalen des Anspruchs 14 gelöst.

Das Bestimmen des wenigstens einen geotechnischen Parameters erfolgt bevorzugt automatisiert.

Bei dem erfindungsgemäßen Verfahren zum Bestimmen wenigstens eines geotechnischen Parameters eines Ausbruchmaterials einer Tunnelbohrmaschine wird das Ausbruchmaterial auf eine Fördervorrichtung zum Transportieren des Ausbruchmaterials aufgegeben. Ein Anprallkörper wird derart in einem Förderstrom des Ausbruchmaterials angeordnet, dass er zumindest teilweise in das Ausbruchmaterial eintaucht, wobei das Ausbruchmaterial in dem Förderstrom eine Strömungsgeschwindigkeit aufweist, die entweder vorgegeben ist oder gemessen wird. Dann wird eine Messgröße, die einer von dem Ausbruchmaterial des Förderstroms auf den Anprallkörper ausgeübten Kraft entspricht, gemessen, wobei eine zeitliche Folge von Messwerten der Messgröße erfasst wird, und aus einem Abschnitt der Folge von Messwerten der wenigstens eine geotechnische Parameter bestimmt wird, indem der Abschnitt der zeitlichen Folge von Messwerten einer Datenverarbeitungseinrichtung eingegeben und von dieser derart verarbeitet wird, dass wenigstens ein dem wenigstens einen geotechnischen Parameter entsprechender Ausgabewert ausgegeben wird, wobei der Verarbeitung der Messwerte entweder die vorgegebene Strömungsgeschwindigkeit zugrunde liegt oder ein Wert für die gemessene Strömungsgeschwindigkeit eingegeben und bei der Verarbeitung der Messwerte berücksichtigt wird.

Die Datenverarbeitungseinrichtung implementiert bevorzugt ein trainiertes neuronales Netzwerk, das mehrere Schichten von Knoten aufweist, wobei sämtlichen Verknüpfungen zwischen den Knoten Gewichte und den Knoten vorgegebene Aktivierungsfunktionen zugeordnet sind, wobei vorab die Gewichte mittels eines Trainingsverfahrens eingestellt worden sind. Aus dem Abschnitt der Folge von Messwerten wird eine Matrix von Eingabedaten gewonnen und die Matrix von Eingabedaten wird einer Eingabeschicht von Knoten des neuronalen Netzwerks eingegeben. Der wenigstens eine dem wenigstens einen geotechnischen Parameter entsprechende Ausgabewert wird von wenigstens einem Knoten einer Ausgabeschicht des neuronalen Netzwerks ausgegeben.

Vorteilhaft ist hierbei, dass direkt am vorhandenen Förderstrom gemessen werden kann und dass der gewünschte geotechnische Parameter, wie beispielsweise das Setzmaß, nach einer sehr kurzen Datenverarbeitungszeit automatisch bereitgestellt wird, was es beispielsweise ermöglicht, die Zugabemenge(n) von Wasser und/oder Schaum und/oder weiteren die rheologischen Eigenschaften des Ausbruchmaterials beeinflussenden Additiven in Abhängigkeit von dem/den bestimmten geotechnischen Parameter(n) einzustellen und somit zu regeln oder das Ausbruchmaterial selbst nach dem geotechnische Parameter zu klassieren.

Bei einer Ausführungsform ist die vorgegebene Aktivierungsfunktion beispielsweise eine reLU-Aktivierungsfunktion (reLU(x) = max(0,x)) oder alternativ - als differenzierbare Approximation der reLU-Aktivierungsfunktion - die Funktion f(x) = In(1 + ex). Den Gewichten werden vor Beginn des Trainingsverfahren Anfangswerte zugewiesen, vorzugsweise kleine zufällig gewählte Anfangswerte, beispielsweise im Intervall (-0,05, 0,05) gleichmäßig verteilte oder um den Wert Null normalverteilte Zufallszahlen. Das Trainingsverfahren verwendet beispielsweise den quadratischen Fehler als Verlustfunktion und ein Gradientenabstiegsverfahren zur Anpassung der Gewichte.

Bei einer Ausführungsform des Verfahrens zum Bestimmen wenigstens eines geotechnischen Parameters könnte der Förderstrom des Ausbruchmaterials durch ein Rohr hindurchgeführt werden, in dem der Anprallkörper, beispielsweise ein in das Rohr hineinragender Stab, montiert ist. Bei einer bevorzugten Ausführungsform des Verfahrens jedoch wird der Anprallkörper in einem offenen Förderstrom des Ausbruchmaterials angeordnet. Vorzugsweise ist dies der oben offene Förderstrom auf einem Förderband.

Bei einer bevorzugten Ausführungsform des Verfahrens zum Bestimmen wenigstens eines geotechnischen Parameters wird eine vorgegebene Strömungsgeschwindigkeit des Förderstroms eingestellt und während des Erfassens der zeitlichen Folge von Messwerten der Messgröße konstant gehalten. Dies vereinfacht die Datenverarbeitung, da keine Messwert-Zeit-Paare, sondern lediglich Messwert-Folgen verarbeitet zu werden brauchen. Voraussetzung ist, dass die eingestellte Strömungsgeschwindigkeit gleich derjenigen Strömungsgeschwindigkeit ist, die bei der Gewinnung von im Trainingsverfahren verwendeten Paarungen aus Messwert-Folge und gemessenem geotechnischen Parameter eingestellt wurde.

Vorzugsweise wird die zeitliche Folge von Messwerten mit einer konstanten Abtastfrequenz erfasst. Auch dies vereinfacht die Datenverarbeitung, da hierbei lediglich ein Wert für die Abtastfrequenz oder die Zeitabstände zwischen den Messpunkten zu verarbeiten oder bei der Verarbeitung anzunehmen ist.

Bei einer bevorzugten Ausführungsform des Verfahrens zum Bestimmen wenigstens eines geotechnischen Parameters entspricht der Abschnitt der zeitlichen Folge von Messwerten einer Messdauer von 0,25 s bis 60 s, vorzugsweise von 0,5 s bis 1 s. Es hat sich in praktischen Versuchen mit üblichen Zusammensetzungen und Strömungsgeschwindigkeiten des Ausbruchmaterials gezeigt, dass die wesentlichen zur Bestimmung des geotechnischen Parameters, insbesondere des Setzmaßes, relevanten Bewegungsabläufe des Anprallkörpers und somit die wesentlichen zu erfassenden Kraftverläufe innerhalb dieser Zeitintervalle stattfinden.

Bei einer Ausführungsform des Verfahrens zum Bestimmen wenigstens eines geotechnischen Parameters werden aus dem Abschnitt der zeitlichen Folge von Messwerten eine vorgegebene Anzahl von charakteristischen Merkmalen berechnet und diese Merkmale als Matrix der Eingabedaten verwendet. Aus dem Abschnitt der zeitlichen Folge von Messwerten, auch als Messsequenz bezeichnet, werden als charakteristische Merkmale beispielsweise Mittelwert, Median, Maximalwert, Minimalwert und/oder Standardabweichung oder auch Fourierkoeffizienten einer diskreten Fouriertransformation berechnet, in einer mehrdimensionalen Matrix angeordnet und normiert der Eingabeschicht übergeben.

Bei einer anderen, einfachen Ausführungsform des Verfahrens zum Bestimmen wenigstens eines geotechnischen Parameters wird der Abschnitt der zeitlichen Folge von Messwerten, die Messsequenz, normiert als eindimensionale Matrix der Eingabedaten verwendet.

Es ist denkbar, dass die Datenverarbeitungseinrichtung das neuronale Netzwerk unter Verwendung einer Spezialhardware implementiert, die eine Vielzahl von Neuronen nachgebildeten Schaltungselementen aufweist. Eine bevorzugte Ausführungsform des Verfahrens zum Bestimmen wenigstens eines geotechnischen Parameters ist jedoch dadurch gekennzeichnet, dass das neuronale Netzwerk von der Datenverarbeitungseinrichtung programmgesteuert simuliert wird (sogenanntes künstliches neuronales Netzwerk). Dies bedeutet, dass die Datenverarbeitungseinrichtung mathematische Operationen ausführt, die die Funktion der Schichten von Neuronen eines neuronalen Netzwerks nachbilden.

Von der Datenverarbeitungseinrichtung können unterschiedliche Arten neuronaler Netzwerke simuliert werden, beispielsweise Feedforward-Netzwerke (unter anderem Mehrschicht-Perzeptrone), faltende Netzwerke oder rekurrente neuronale Netzwerke. Bei einer bevorzugten Weiterbildung dieses Verfahrens zum Bestimmen wenigstens eines geotechnischen Parameters wird von der Datenverarbeitungseinrichtung ein neuronales Feedforward-Netzwerk mit einem eindimensionalen Faltungsnetzwerk simuliert. Durch die Verwendung von Faltungsnetzwerken (Convolutional Neural Networks - CNN) wird die Menge der auszuführenden Rechenoperationen gegenüber einem vollständig verknüpften Netzwerk erheblich reduziert. Zugleich haben lokal nahe Neuronen, somit im vorliegenden Fall zeitlich nahe Messwerte einer Messequenz, einen größeren Einfluss auf die Neuronen einer nachfolgenden Schicht.

Bei einem gegenwärtig bevorzugten Ausführungsbeispiel des Verfahrens zum Bestimmen wenigstens eines geotechnischen Parameters wird von der Datenverarbeitungseinrichtung ein neuronales Faltungsnetzwerk mit einer ersten Faltungsschicht mit nachfolgender Pooling-Schicht und einer nachfolgenden zweiten Faltungsschicht mit mehreren nachfolgenden vollständig verknüpften Schichten simuliert. Es zeigte sich, dass mit einer derartigen Ausbildung des neuronalen Netzwerks sich einerseits eine Überanpassung vermeiden lässt und anderseits gute Vorhersagen erzielt werden können.

Bevorzugte Ausführungsformen des Verfahrens zum Bestimmen wenigstens eines geotechnischen Parameters sind dadurch gekennzeichnet, dass vor dem Eingeben des Abschnitts der zeitlichen Folge von Messwerten zum Bestimmen des wenigstens einen geotechnischen Parameters die Gewichte in dem Trainingsverfahren eingestellt werden, indem
a) für eine vorgegebene Auswahl einer Vielzahl von Ausbruchmaterial-Proben unterschiedlicher Zusammensetzungen mit unterschiedlichen geotechnischen Parametern jeweils:
   a1) der wenigstens eine geotechnische Parameter, beispielsweise das Setzmaß ("slump"), der Ausbruchmaterial-Probe gemessen oder bestimmt und als Ziel-Parameterwert gespeichert wird,
   a2) der Anprallkörper derart in einem Förderstrom der Ausbruchmaterial-Probe angeordnet wird, dass er zumindest teilweise in das Ausbruchmaterial eintaucht, wobei das Ausbruchmaterial in dem Förderstrom eine vorgegebene Strömungsgeschwindigkeit aufweist, und eine Messgröße, die einer von dem Ausbruchmaterial des Förderstroms der Ausbruchmaterial-Probe auf den Anprallkörper ausgeübten Kraft entspricht, gemessen wird, wobei eine zeitliche Folge von Messwerten der Messgröße erfasst wird,
   a3) Paarungen aus jeweils einem Abschnitt der Folge von Messwerten in Zuordnung zu dem Ziel-Parameterwert gespeichert werden,
b) in dem von der Datenverarbeitungseinrichtung implementierten neuronalen Netzwerk den Gewichten Anfangswerte zugewiesen werden,
c) für eine Paarung aus einem Abschnitt der Folge von Messwerten und zugeordnetem Ziel-Parameterwert:
   c1) aus dem Abschnitt der Folge von Messwerten der wenigstens eine geotechnische Parameter bestimmt wird, indem der Abschnitt der zeitlichen Folge von Messwerten von der das neuronale Netzwerk implementierenden Datenverarbeitungseinrichtung derart verarbeitet wird, dass wenigstens ein dem wenigstens einen geotechnischen Parameter entsprechender Ausgabewert ausgegeben wird,
   c2) dann die Differenz zwischen dem Ausgabewert und dem zugeordneten Ziel-Parameterwert bestimmt wird und
   c3) in Abhängigkeit von der Differenz die Gewichte geändert werden,

wobei die Schritte c1) bis c3) für eine nächste Paarung aus einem Abschnitt der Folge von Messwerten und zugeordnetem Ziel-Parameterwert wiederholt werden, wobei die Auswahl der Paarungen in zufälliger Reihenfolge erfolgt,
wobei die Schritte c1) bis c3) für einen vorgegebenen Anteil der im Schritt a) gespeicherten Paarungen ausgeführt werden, der als Trainingsmenge dient.

Eine Anwendung des genannten Verfahrens zum Bestimmen wenigstens eines geotechnischen Parameters ist erfindungsgemäß ein Verfahren zum Einstellen der Konsistenz eines konditionierten Ausbruchmaterials einer Erddruckschild-Tunnelbohrmaschine, bei welchem dem Ausbruchmaterial zu dessen Konditionierung in der Abbaukammer ein Konditionierungsmittel, beispielsweise ein Tensid-Schaum, zugemischt wird, das so konditionierte Ausbruchmaterial mit Fördervorrichtungen aus der Abbaukammer gefördert wird, wobei in einem auf einer der Fördervorrichtungen ausgebildeten offenen Förderstrom wenigstens ein geotechnischer Parameter, vorzugsweise ein Setzmaß, nach einem der oben genannten Verfahren automatisiert bestimmt wird, und in Abhängigkeit von dem so bestimmten geotechnischen Parameter die Menge des zuzumischenden Konditionierungsmittels geregelt wird, bevorzugt indem in Abhängigkeit von einer Differenz zwischen dem so bestimmten geotechnischen Parameter und einem Sollwert des geotechnischen Parameters die Menge des zuzumischenden Konditionierungsmittels erhöht oder abgesenkt wird.

Eine Anwendung des genannten Verfahrens zum Bestimmen wenigstens eines geotechnischen Parameters ist erfindungsgemäß ein Verfahren zum Klassieren eines Ausbruchmaterials einer Tunnelbohrmaschine, wobei das konditionierte Ausbruchmaterial mit wenigstens einer Fördervorrichtungen aus der Abbaukammer gefördert wird, wobei in einem auf einer der Fördervorrichtungen ausgebildeten offenen Förderstrom wenigstens ein geotechnischer Parameter, vorzugsweise ein Setzmaß, nach einem der oben genannten Verfahren automatisiert bestimmt wird, und das Ausbruchmaterial in Abhängigkeit von dem bestimmten geotechnischen Parameter klassiert wird, indem vorzugsweise in Abhängigkeit von einer Differenz zwischen dem so bestimmten geotechnischen Parameter und einem Sollwert des geotechnischen Parameters das Ausbruchmaterial klassiert wird.

Die erfindungsgemäße Vorrichtung zum Bestimmen wenigstens eines geotechnischen Parameters eines in einer Tunnelbohrmaschine anfallenden Ausbruchmaterials umfasst (a) eine Fördervorrichtung zum Transportieren des Ausbruchmaterials derart, dass in einem offenen Förderkanal ein Förderstrom des Ausbruchmaterials mit einer Strömungsgeschwindigkeit ausgebildet wird, wobei die Fördervorrichtung eine Einrichtung zum Einstellen einer vorgegebenen Strömungsgeschwindigkeit und/oder eine Einrichtung zum Messen der Strömungsgeschwindigkeit aufweist, (b) einen in dem offenen Förderkanal angeordneten Anprallkörper, der zumindest teilweise in den Förderstrom des Ausbruchmaterials eintaucht, (c) eine mit dem Anprallkörper verbundene Messvorrichtung zum Messen einer Messgröße, die einer von dem Ausbruchmaterial des Förderstroms auf den Anprallkörper ausgeübten Kraft entspricht, (d) eine mit der Messvorrichtung gekoppelte Vorrichtung zum Erfassen von Abtastwerten der Messgröße, zum Umwandeln der Abtastwerte der Messgröße in digitale Messwerte und zum Ausgeben einer zeitlichen Folge von digitalen Messwerten, (e) eine Speichervorrichtung zum Zwischenspeichern der Folge von digitalen Messwerten und (f) eine mit der Speichervorrichtung gekoppelte Datenverarbeitungseinrichtung, die so konfiguriert ist, dass sie den Abschnitt der Folge von digitalen Messwerten verarbeitet und wenigstens einen dem wenigstens einen geotechnischen Parameter entsprechenden Ausgabewert ausgibt.

Dabei ist vorteilhaft, dass die Datenverarbeitungseinrichtung so konfiguriert ist, dass sie aus dem Abschnitt der Folge von digitalen Messwerten eine Matrix von Eingabedaten erzeugt und verarbeitet und wenigstens einen dem wenigstens einen geotechnischen Parameter entsprechenden Ausgabewert ausgibt und bei der Verarbeitung entweder die eingestellte vorgegebene Strömungsgeschwindigkeit oder einen gemessenen Wert der Strömungsgeschwindigkeit berücksichtigt, wobei die Datenverarbeitungseinrichtung ein trainiertes neuronales Netzwerk implementiert, das mehrere Schichten von Knoten aufweist, wobei sämtlichen Verknüpfungen zwischen den Knoten Gewichte und den Knoten vorgegebene Aktivierungsfunktionen zugeordnet sind, wobei vorab die Gewichte mittels eines Trainingsverfahrens eingestellt worden sind, wobei die Matrix von Eingabedaten einer Eingabeschicht von Knoten des neuronalen Netzwerks eingegeben und der wenigstens eine dem wenigstens einen geotechnischen Parameter entsprechende Ausgabewert an wenigstens einem Knoten einer Ausgabeschicht des neuronalen Netzwerks ausgegeben wird.

Der Anprallkörper kann ein beliebig gestalteter Körper sein, der so in den Förderstrom des Ausbruchmaterials eingebracht wird, dass eine von den rheologischen Eigenschaften des Ausbruchmaterials abhängige Kraft auf den Anprallkörper einwirkt. Der Anprallkörper kann beispielsweise ein in den Förderstrom hineinragender Stab mit rundem Querschnitt sein. Bei einem bevorzugten Ausführungsbeispiel der Vorrichtung zum Bestimmen wenigstens eines geotechnischen Parameters ist der Anprallkörper als Kugel ausgebildet. Ein solcher Anprallkörper hat den Vorteil, dass er nicht in einer bestimmten Orientierung in dem Strom angeordnet zu werden braucht. Ein pflugähnlicher Körper ist ebenfalls eine vorteilhafte Ausführungsform.

Bei einer bevorzugten Vorrichtung zum Bestimmen wenigstens eines geotechnischen Parameters ist der Anprallkörper eine Stahlkugel mit einem Gewicht von 500 g bis 2000 g, vorzugsweise 800 g bis 1000 g, ist. Eine solche Stahlkugel hat beispielsweise einen Durchmesser von etwa 6 cm und ein Gewicht von etwa 900 g. Eine derart dimensionierte Stahlkugel ist bei den üblichen Strömungsgeschwindigkeiten (im Bereich zwischen 1 m/s und 3 m/s) und Zusammensetzungen des Förderstroms einerseits ausreichend schwer, so dass sie nicht übermäßig auf der Oberfläche des Förderstroms des Ausbruchmaterials "hüpft", andererseits aber nicht zu schwer, so dass sie vom Förderstrom nahezu unbeeinflusst in konstanter Position verharrt.

Der Anprallkörper, beispielsweise in Form einer Kugel, kann an einer festen Halterung, beispielsweise einem Stab, montiert sein und in den Förderstrom hineinragen. Beispielsweise könnte ein Dehnmessstreifen der Messvorrichtung die kraftabhängige Durchbiegung eines solchen Stabes aufnehmen.

Bei einer bevorzugten Weiterbildung der Vorrichtung zum Bestimmen wenigstens eines geotechnischen Parameters jedoch ist ein kugelförmiger Anprallkörper an einem schwenkbar aufgehängten Stab oder einem Zugseil befestigt und ist ein Sensor zum Erfassen der Messgröße mit dem Stab beziehungsweise dem Zugseil gekoppelt, wobei die Messgröße einer von dem Stab beziehungsweise dem Zugseil aufgenommenen Zugkraft entspricht. Die Aufhängung des Anprallkörpers an einem schwenkbar aufgehängten Stab oder einem Zugseil ist von Vorteil, weil sie ein Ausweichen des Anprallkörpers bei Antreffen eines relativ großen im Ausbruchmaterialstrom mitgeführten Körpers (z.B. Steins) ermöglicht.

Bei einer bevorzugten Vorrichtung zum Bestimmen wenigstens eines geotechnischen Parameters ist die Datenverarbeitungseinrichtung so konfiguriert, dass sie ein neuronales Feedforward-Netzwerk in Form eines eindimensionalen Faltungsnetzwerks simuliert. Vorzugsweise ist die Datenverarbeitungseinrichtung dabei so konfiguriert, dass sie ein neuronales eindimensionales Faltungsnetzwerk mit einer ersten Faltungsschicht mit nachfolgender Pooling-Schicht und einer nachfolgenden zweiten Faltungsschicht mit mehreren nachfolgenden vollständig verknüpften Schichten simuliert. Dies hat die oben im Kontext der entsprechenden bevorzugten Ausführungsform des Verfahrens genannten Vorteile.

Vorteilhafte und/oder bevorzugte Weiterbildungen der Erfindung sind in den Unteransprüchen gekennzeichnet.

Im Folgenden wird die Erfindung anhand eines in den Zeichnungen dargestellten bevorzugten Ausführungsbeispiels näher beschrieben. In den Zeichnungen zeigen:
Figur 1 eine Prinzipskizze der Messanordnung einer bevorzugten Vorrichtung zum Bestimmen wenigstens eines geotechnischen Parameters,
Figur 2 eine beispielhafte Darstellung einer Messreihe und einen eine Messsequenz darstellenden Abschnitt der Messreihe, und
Figur 3 eine Prinzipskizze zur Veranschaulichung des von einer Datenverarbeitungseinrichtung simulierten neuronalen Netzwerks.

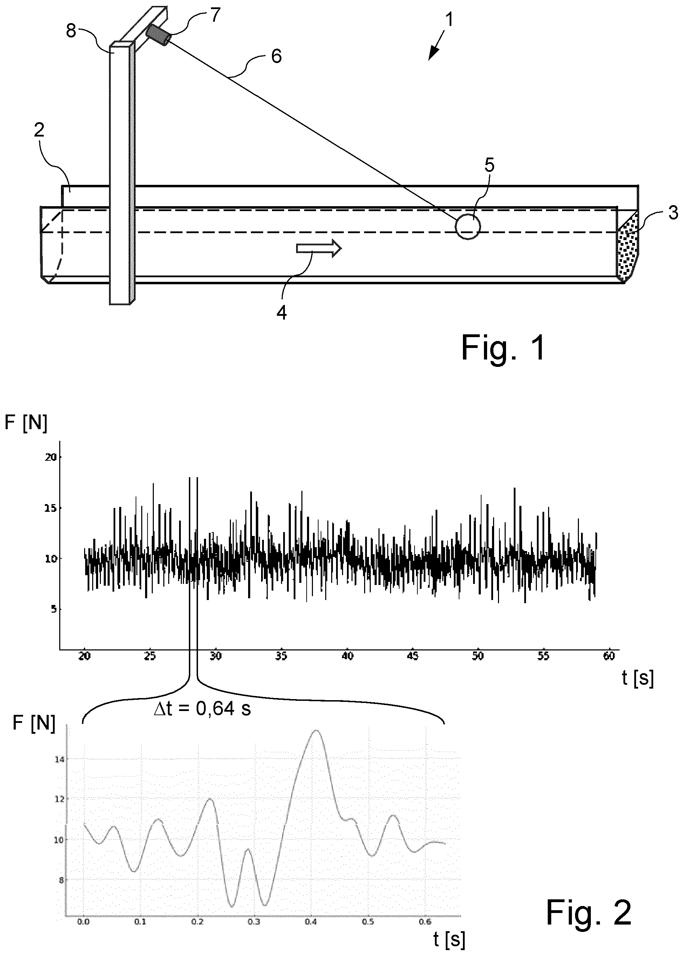

Figur 1 zeigt schematisch eine bei der erfindungsgemäßen Vorrichtung zum Ermitteln wenigstens eines geotechnischen Parameters, insbesondere eines Index-Parameters wie dem Setzmaß, verwendete rheologische Messanordnung 1.

Die Messanordnung 1 dient dazu, einen geotechnischen Parameter, wie insbesondere das Setzmaß, in einem Förderstrom 3 zu bestimmen, wobei sich der Förderstrom 3 mit einer vorgegebenen Geschwindigkeit in einem Förderkanal 2 bewegt. Die Bewegung des Förderstroms 3 ist durch den Pfeil 4 angezeigt.

Beispielsweise befindet sich der Förderkanal 2 auf einem Förderband einer Tunnelbohrmaschine. Die Geschwindigkeit des Förderstroms 3 liegt im Bereich zwischen 1 und 3 m/s, vorzugsweise im Bereich 1,8 m/s bis 2,5 m/s.

Ein an einem Zugseil 6 aufgehängter Anprallkörper, hier bevorzugt in Form einer Stahlkugel 5, taucht zumindest teilweise in den Förderstrom 3 ein. An der Aufhängung des Zugseils 6 an einer Aufständerung mit Traverse 8 befindet sich ein Kraftaufnehmer 7, der die an dem Zugseil 6 wirkende Kraft misst. Bei typischen Ausbruchmaterialien, die sich mit einer Geschwindigkeit zwischen 1,8 m/s bis 2,5 m/s in dem Förderkanal 2 bewegen, wird vorzugsweise eine Stahlkugel von 6 cm Durchmesser mit einem Gewicht von etwa 900 g verwendet.

Der Kraftaufnehmer 7 ist mit einer (in Figur 1 nicht dargestellten) Auswerteeinrichtung zum Auswerten des Sensorausgangssignals verbunden, die das Ausgangssignal des Kraftaufnehmers 7 mit einer vorgegebenen Abtastfrequenz abtastet und die so erfassten Abtastwerte ausgibt und/oder zwischenspeichert. Bei der Versuchsanordnung liegt die Abtastfrequenz beispielweise bei 200 Hz, so dass im Abstand von jeweils 5 ms ein Kraft-Abtastwert erfasst und gespeichert wird.

Figur 2 zeigt im oberen Diagramm die bei einer Versuchsanordnung jeweils erfasste Messreihe, bei der die Abtastwerte für eine Dauer beispielsweise von etwa 60 Sekunden erfasst wurden, wobei beispielsweise die in den ersten etwa 20 Sekunden erfassten Abtastwerte, die während des Anfahrens der Versuchsanordnung gewonnen wurden, verworfen wurden.

Die Messreihe der im Abstand von jeweils 5 ms gewonnenen Kraft-Messwerte wurde in 61 Messsequenzen von beispielsweise jeweils 128 Abtastwerten und somit von etwa 0,64 s Dauer unterteilt.

Das untere Diagramm der Figur 2 stellt beispielhaft einen Sensorsignalverlauf während des Zeitintervalls von 0,64 s dar, in dem die 128 Abtastwerte gewonnen wurden. Die Wahl von 128 Abtastwerten pro Sequenz ist auch deshalb von Vorteil, weil eine Zweierpotenz die rechnerische Behandlung, beispielsweise bei einer diskreten Fouriertransformation zum Gewinnen von charakteristischen Merkmalen aus der Messsequenz, erleichtert.

Die jeweils 128 Abtastwerte einer Messsequenz wurden den Knoten einer Eingabe-Schicht eines neuronalen Netzwerkes eingegeben, nachdem das neuronale Netzwerk in einer nachfolgend näher beschriebenen Weise trainiert worden ist. Ein korrekt trainiertes neuronales Netzwerk gibt dann einen der eingespeisten Messsequenz von 128 Abtastwerten entsprechenden Wert für einen geotechnischen Parameter aus, im bevorzugten Ausführungsbeispiel einen Wert für das Setzmaß ("Siump-Wert"). Bei alternativen Ausführungsformen können auch andere und mehrere geotechnische Parameterwerte ausgegeben werden.

Für ein Training, d. h. ein überwachtes Lernen des neuronalen Netzwerks ist es erforderlich, Trainingsdaten bereitzustellen, die Paarungen aus jeweils einer Messequenz von 128 Kraft-Werten und einem zugehörigen Wert des gemessenen geotechnischen Parameters, d. h. im vorliegenden Fall des Setzmaßes, umfassen.

Zum Erzeugen der Trainingsdaten für das neuronale Netzwerk wurde bei einer bevorzugten Ausführungsform wie folgt vorgegangen. Zunächst wurden Proben von Ausbruchmaterialien unterschiedlicher Zusammensetzung und Konsistenz bereitgestellt, hier beispielsweise 13 Proben, die durch eine Reihe unterschiedlicher Setzmaße im relevanten Bereich gekennzeichnet waren.

Zu jeder der Proben wurde der geotechnische Parameter, im vorliegenden Fall beispielsweise das Setzmaß, messtechnisch erfasst. Die Proben wurden sukzessive in eine Versuchsanordnung gebracht, in der ein Förderstrom der jeweils eingebrachten Probe auf eine vorgegebene Fördergeschwindigkeit von 1,8 m/s oder 2,5 m/s gebracht wurde.

Ein Anprallkörper in Form einer an einem Zugseil befestigten Stahlkugel wurde in den Förderstrom eingebracht und nach dem Einschwingen der Anordnung bei der vorgegebenen Geschwindigkeit eine Messreihe von Kraft-Abtastwerten erfasst und gespeichert, wie sie beispielhaft in der oberen Abbildung der Figur 2 dargestellt ist.

Für jede der beispielsweise 13 Proben wurden Messreihen der dargestellten Art erzeugt, hier bevorzugt drei Messreihen. Jede der drei Messreihen wiederum wurde in 61 Messsequenzen von jeweils 128 Abtastwerten unterteilt, so dass für jedes der 13 Probe-Ausbruchmaterialien 183 Messsequenzen, also insgesamt 13 x 183 = 2379 Messsequenzen erzeugt und gespeichert wurden. Der gemessene geotechnische Parameter, das Setzmaß, einer Probe wurde in Zuordnung zu der bei dieser Probe erfassten Messequenz gespeichert. Somit wurden 2379 Paarungen von Messsequenzen mit 128 Kraft-Werten und einem zugehörigen Setzmaß-Wert gespeichert.

Aus der Gesamtzahl von 2379 Messequenz-Setzmaß-Paarungen wurden etwa 2000 Paarungen zufällig als Trainings-Paarungen zum Trainieren des neuronalen Netzwerks ausgewählt. Eine Restmenge von etwa 300 zufällig ausgewählten Paarungen dient als Validierungsdatensätze zur Prüfung der Qualität und Eignung des trainierten neuronalen Netzwerks.

Das neuronale Netzwerk wurde trainiert, indem als Verlustfunktion der quadratische Fehler und das Gradientenabstiegsverfahren verwendet wurden.

Die Gewichte des neuronalen Netzwerks wurden zunächst auf einen beliebig kleinen Wert voreingestellt. Dann wurden die 128 Abtastwerte, die den gemessenen Kraftverlauf innerhalb eines Zeitintervalls von 0,64 s wiedergeben, einer Eingabe-Schicht des neuronalen Netzwerks eingegeben und auf der Basis der voreingestellten Gewichte ein Ausgabe-Wert an einer Ausgabe-Schicht gewonnen, der dem geotechnischen Parameter entsprechen soll. Dann wurde die Differenz zwischen dem durch das neuronale Netzwerk ermittelten geotechnischen Parameter und dem für die Probe gemessenen tatsächlichen Wert des geotechnischen Parameters bestimmt. In Abhängigkeit von diesen Differenzen zwischen den vom neuronalen Netzwerk ausgegebenen Parameterwerten und den gemessenen Ist-Werten der Proben wurden in bekannter Weise die Gewichte angepasst, wobei dies für sämtliche Paarungen aus Kraft-Abtastwerten einer Messsequenz und dem für die zugehörige Probe gemessenen geotechnischen Parameter (Setzmaß) wiederholt wurden. Hierbei wurden die Trainings-Paarungen in zufälliger Reihenfolge eingegeben.

Die Details des unter Verwendung einer Verlustfunktion des quadratischen Fehlers und des Gradientenabstiegsverfahrens durchgeführten überwachten Lernens sind einem Fachmann auf dem Gebiet der neuronalen Netzwerke bekannt und brauchen hier nicht im Detail beschrieben zu werden.

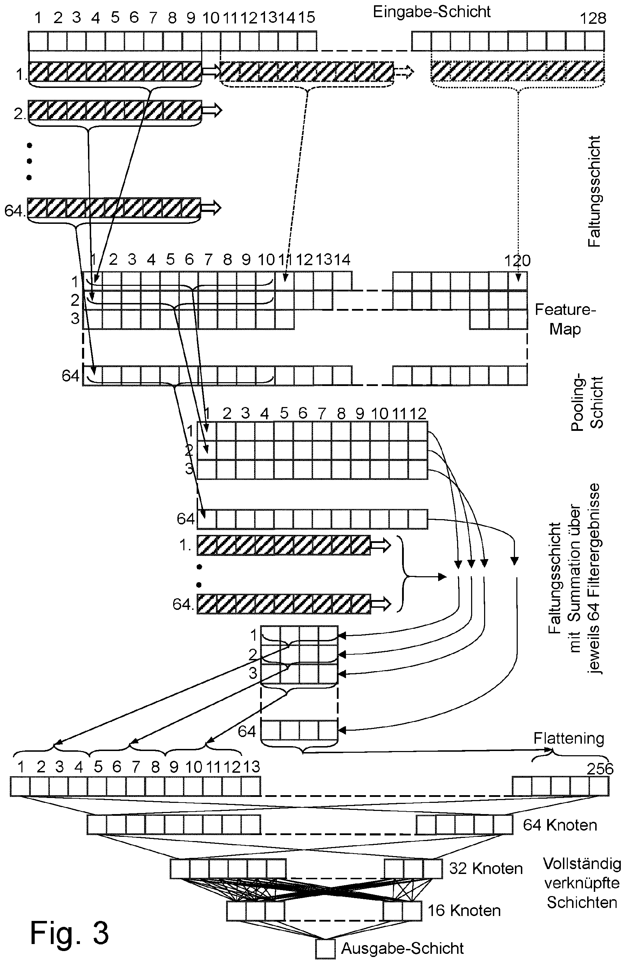

Nachfolgend wird die Struktur eines beispielhaften neuronalen Netzwerks beschrieben, das auch bei der Versuchsanordnung verwendet wurde. Figur 3 zeigt eine schematische Darstellung des neuronalen Netzwerks, das durch die Berechnungen der Datenverarbeitungseinrichtung simuliert wird, bei denen aus einer Messsequenz von 128 Kraft-Abtastwerten ein Wert für einen geotechnischen Parameter (hier ein Setzmaß) bestimmt wird. Dieses künstliche neuronale Netzwerk umfasst zunächst die in der obersten Zeile der Figur 3 dargestellten 128 Knoten einer Eingabe-Schicht. Die darunter dargestellten Berechnungen simulieren eine Faltungsschicht (Convolution Layer).

Bei einer Faltung wird ein Faltungsfenster (auch als Filter, Kernel oder Faltungskern bezeichnet) einer vorgegebenen Breite von hier neun Gewichtswerten, das in Figur 3 schraffiert dargestellt ist, mit den Werten von 9 Eingabe-Knoten verknüpft, wobei jeweils ein Gewichtswert mit dem Wert eines Knotens multipliziert und die so gebildeten neun Produkte summiert werden (Vektorskalarprodukt). Das Ergebnis wird mit einer Aktivierungsfunktion bewertet und in einem Speicherplatz einer sogenannten Feature-Map gespeichert.

Anschließend wird das Faltungsfenster um einen Knoten der Eingabe-Schicht weitergerückt (nach rechts in Figur 3), so dass die neun Gewichtswerte des Faltungsfensters dann mit den Werten der Knoten 2 - 10 der Eingabe-Schicht verknüpft werden (jeweils multipliziert und die Ergebnisse summiert).

In Figur 3 ist in der Zeile unter der Eingabe-Schicht ein erstes Faltungsfenster der Breite 9 zunächst unterhalb der Knoten 1 - 9 der Eingabe-Schicht dargestellt, wobei das Ergebnis dieses Skalarprodukts in dem ersten Feld in der ersten Zeile der unter den Faltungsfenstern dargestellten Feature-Map gespeichert wird. Dies ist durch einen das erste Faltungsfenster mit dem Ergebnisfeld verbindenden Pfeil dargestellt.

Rechts neben dem ersten Faltungsfenster ist in gestrichelter Darstellung ein um elf Schritte weitergerücktes erstes Faltungsfenster dargestellt, was veranschaulichen soll, dass in dieser Position die neun Gewichte des ersten Faltungsfensters mit den Werten der Knoten 11 - 19 der Eingabe-Schicht verknüpft werden, wobei das Ergebnis in dem elften Feld der ersten Zeile der Feature-Map gespeichert wird.

Schließlich ist ganz rechts eine weitere gestrichelte Darstellung des ersten Faltungsfensters gezeigt, das so positioniert ist, dass seine Gewichtswerte mit den Knoten 120 -128 der Eingabe-Schicht verknüpft werden, wobei das Ergebnis in dem 120. Feld der ersten Zeile der Feature-Map gespeichert wird, was wiederum durch einen entsprechenden Pfeil angezeigt wird.

Bei dem in Figur 3 dargestellten bevorzugten Ausführungsbeispiel werden in der ersten Faltungsschicht insgesamt 64 verschiedene Faltungsfenster oder Filter verwendet, was durch die Nummerierung 1, 2 und 64 links neben den Filterblöcken veranschaulicht ist. Dementsprechend werden durch die gesamten Faltungsoperationen Ergebnisse erzeugt, die in einer Feature-Map mit jeweils 120 Feldern in 64 Zeilen gespeichert werden. Diese Operationen simulieren eine erste eindimensionale Faltungsschicht des neuronalen Netzwerks. Die Faltungsschicht ist eindimensional, wobei über eine eindimensionale Eingabe-Schicht ein eindimensionales Faltungsfenster bewegt wird.

Vor ihrer Weiterverarbeitung werden die in der Feature-Map gespeicherten Werte bevorzugt noch mit einer sogenannten Aktivierungsfunktion bewertet. Diese Bewertung erfolgt entweder bereits bei ihrer Berechnung (Skalarprodukt) vor Speicherung in die Feature-Map oder nachträglich für die gesamte berechnete Feature-Map. Beide Vorgehensweisen sind bei einem simulierten neuronalen Netzwerk äquivalent.

Bei dem hier dargestellten bevorzugten Ausführungsbeispiel wird eine sogenannte reLU-Aktivierungsfunktion verwendet, d. h. reLU(x) = Max(0,x). Dies bedeutet, dass sämtliche negativen Werte durch Null ersetzt werden, während die positiven Werte unverändert bleiben.

Um die Berechnungszeit und den Rechenaufwand zu reduzieren, wird die in der Feature-Map gespeicherte Datenmenge von 120 x 64 Elementen bevorzugt in einer sogenannten Pooling-Schicht reduziert, indem aus jeweils einer vorgegebenen Anzahl von Elementen der Feature-Map ein neues Element gewonnen wird. Bei dem in Figur 3 dargestellten bevorzugten Ausführungsbeispiel wird aus jeweils 10 Elementen einer Zeile der Feature-Map ein Mittelwert gebildet und dieser (in einer weiteren Knotenschicht) zwischengespeichert. Diese Mittelwertbildung von jeweils 10 Elementen ist durch eine geschweifte Klammer mit einem Pfeil beispielhaft für die ersten 10 Elemente der ersten 1., 2. und 64. Zeile dargestellt.

Insgesamt resultieren 12 x 64 = 768 Mittelwerte. Aufgrund der Mittelwertbildung wird diese Operation auch als Average-Pooling bezeichnet. Es wäre auch ein neuronales Netzwerk denkbar, das in seiner Pooling-Schicht anstelle eines Average-Pooling ein sogenanntes Max-Pooling verwendet, bei dem von der vorgegebenen Anzahl von Ausgangswerten jeweils nur der Maximalwert ausgewählt wird (auch als Subsampling bezeichnet).

Auf dieses Pooling folgt eine weitere Faltungsschicht, wie es in Figur 3 dargestellt ist. Bei der weiteren Faltung werden wiederum 64 verschiedene Faltungsfenster mit jeweils einer Breite von 9 nacheinander über die Zeilen der Ergebnisse des Pooling von jeweils 12 Elementen bewegt, wobei bei jedem Bewegen eines Faltungsfensters der Breite 9 über eine Zeile von 12 Elementen vier Ergebnis-Elemente gebildet werden. Hierbei werden die 64 verschiedenen Faltungsfenster oder Filter nacheinander (oder auch parallel) über eine Zeile von 12 Elementen bewegt, wobei die ersten, die zweiten, die dritten und die vierten Ergebniselemente der 64 Filter jeweils aufsummiert werden.

Dies wird für jede der 64 Zeilen der Ergebnisse des Pooling wiederholt, so dass insgesamt eine Matrix von 4 x 64 Elementen gebildet wird, wie es in der Figur 3 dargestellt ist. Auch die Ergebnisse dieser Faltungsoperationen werden mit einer Aktivierungsfunktion, vorzugsweise wiederum einer reLU-Aktivierungsfunktion bewertet. Die 4 x 64 bewerteten Faltungsergebnisse werden dann bei einem sogenannten Flattening in einen Vektor von 256 Elementen umsortiert, wobei dieser Vektor in Figur 3 als Knotenschicht von 256 Knoten dargestellt ist.

Es folgen drei weitere Schichten von Knoten des neuronalen Netzwerks, die 64, 32 bzw. 16 Knoten aufweisen. Diese vier verdeckten Knoten-Schichten ("hidden layer") mit 256, 64, 32 bzw. 16 Knoten, die in Figur 3 unten dargestellt sind, sind vollständig verknüpfte Schichten ("fully connected layer"), d. h., jeder Knoten ist mit jedem Knoten der nachfolgenden Schicht verknüpft, wobei diese Verknüpfungen in Figur 3 nicht vollständig dargestellt sind.

Jeder Verknüpfung zwischen zwei Knoten ist ein Gewicht zugeordnet. Jeder der in Figur 3 dargestellten Knoten implementiert außerdem wieder eine Aktivierungsfunktion, vorzugsweise die reLU-Aktivierungsfunktion. Auf die letzte Schicht mit 16 Knoten folgt eine Ausgabeschicht, die bei dem in Figur 3 dargestellten Beispiel lediglich einen Ausgabe-Knoten zum Ausgeben eines Werts, hier beispielsweise für das Setzmaß, umfasst. Diesem Knoten ist keine Aktivierungsfunktion zugeordnet.

Im Verlauf des überwachten Lernens sind sowohl den jeweils neun Gewichten der 64 Faltungsfenster der ersten Faltungsschicht als auch den jeweils neun Gewichten der 64 Fenster der zweiten Faltungsschicht sowie den 256 x 64 + 64 x 32 + 32 x 16 + 16 = 18.960 Gewichten der vollständig verknüpften Schichten sowie den Gewichten zusätzlich vorhandener Bias-Neuronen Anfangswerte zuzuweisen und dann diese Werte durch ein geeignetes Verfahren anzupassen.

Selbstverständlich sind viele alternative Konfigurationen für geeignete neuronale Netzwerke denkbar. Beispielsweise könnten anstelle von 64 Filtern der Faltungsschichten auch nur 16 Filter verwendet und gleichzeitig auf das Pooling nach der ersten Faltungsschicht verzichtet werden. Bei der Netzkonfiguration ist stets abzuwägen, dass die Anzahl der erlernbaren Parameter (Gewichte) nicht zu gering ist, um gute Vorhersageergebnisse zu erzielen, aber auch nicht zu hoch sein sollte, weil es dann zu einer Überanpassung des neuronalen Netzwerks kommen kann. Die hier anhand von Figur 3 beschriebene Struktur eines neuronalen Netzwerks ist das Ergebnis einer Vielzahl von Versuchen mit unterschiedlichen neuronalen Netzwerken.

## Patentansprüche

1. Verfahren zum Bestimmen wenigstens eines geotechnischen Parameters eines Ausbruchmaterials einer Tunnelbohrmaschine, wobei
das Ausbruchmaterial auf eine Fördervorrichtung (2) zum Transportieren des Ausbruchmaterials aufgegeben wird,
ein Anprallkörper (5) derart in einem Förderstrom (3) des Ausbruchmaterials angeordnet wird, dass er zumindest teilweise in das Ausbruchmaterial eintaucht, wobei das Ausbruchmaterial in dem Förderstrom (3) eine Strömungsgeschwindigkeit aufweist, die entweder vorgegeben ist oder gemessen wird,
eine Messgröße, die einer von dem Ausbruchmaterial des Förderstroms (3) auf den Anprallkörper (5) ausgeübten Kraft entspricht, gemessen wird, wobei eine zeitliche Folge von Messwerten der Messgröße erfasst wird, und
aus einem Abschnitt der Folge von Messwerten der wenigstens eine geotechnische Parameter bestimmt wird, indem der Abschnitt der zeitlichen Folge von Messwerten einer Datenverarbeitungseinrichtung eingegeben und von dieser derart verarbeitet wird, dass wenigstens ein dem wenigstens einen geotechnischen Parameter entsprechender Ausgabewert ausgegeben wird, wobei der Verarbeitung der Messwerte entweder die vorgegebene Strömungsgeschwindigkeit zugrunde liegt oder ein Wert für die gemessene Strömungsgeschwindigkeit eingegeben und bei der Verarbeitung der Messwerte berücksichtigt wird.

2. Verfahren zum Bestimmen wenigstens eines geotechnischen Parameters nach Anspruch 1, **dadurch gekennzeichnet, dass** der Anprallkörper in einem offenen Förderstrom des Ausbruchmaterials angeordnet wird.

3. Verfahren zum Bestimmen wenigstens eines geotechnischen Parameters nach Anspruch 1 oder 2, **dadurch gekennzeichnet, dass** eine vorgegebene Strömungsgeschwindigkeit des Förderstroms eingestellt und während des Erfassens der zeitlichen Folge von Messwerten der Messgröße konstant gehalten wird.

4. Verfahren zum Bestimmen wenigstens eines geotechnischen Parameters nach einem der Ansprüche 1 - 3, **dadurch gekennzeichnet, dass** die zeitliche Folge von Messwerten mit einer konstanten Abtastfrequenz erfasst wird.

5. Verfahren zum Bestimmen wenigstens eines geotechnischen Parameters nach einem der Ansprüche 1 - 4, **dadurch gekennzeichnet, dass** der Abschnitt der zeitlichen Folge von Messwerten einer Messdauer von 0,25 s bis 60 s, vorzugsweise von 0,5 s bis 1 s, entspricht.

6. Verfahren zum Bestimmen wenigstens eines geotechnischen Parameters nach einem der Ansprüche 1 - 5, **dadurch gekennzeichnet, dass** die Datenverarbeitungseinrichtung ein trainiertes neuronales Netzwerk implementiert, das mehrere Schichten von Knoten aufweist, wobei sämtlichen Verknüpfungen zwischen den Knoten Gewichte und den Knoten vorgegebene Aktivierungsfunktionen zugeordnet sind, wobei vorab die Gewichte mittels eines Trainingsverfahrens eingestellt worden sind,
wobei aus dem Abschnitt der Folge von Messwerten eine Matrix von Eingabedaten gewonnen wird und die Matrix von Eingabedaten einer Eingabeschicht von Knoten des neuronalen Netzwerks eingegeben wird und
wobei der wenigstens eine dem wenigstens einen geotechnischen Parameter entsprechende Ausgabewert von wenigstens einem Knoten einer Ausgabeschicht des neuronalen Netzwerks ausgegeben wird.

7. Verfahren zum Bestimmen wenigstens eines geotechnischen Parameters nach Anspruch 6, **dadurch gekennzeichnet, dass** aus dem Abschnitt der zeitlichen Folge von Messwerten eine vorgegebene Anzahl von charakteristischen Merkmalen berechnet werden und diese Merkmale als Matrix der Eingabedaten verwendet werden.

8. Verfahren zum Bestimmen wenigstens eines geotechnischen Parameters nach Anspruch 6, **dadurch gekennzeichnet, dass** der Abschnitt der zeitlichen Folge von Messwerten normiert als eindimensionale Matrix der Eingabedaten verwendet wird.

9. Verfahren zum Bestimmen wenigstens eines geotechnischen Parameters nach einem der Ansprüche 6 - 8, **dadurch gekennzeichnet, dass** von der Datenverarbeitungseinrichtung programmgesteuert ein neuronales Netzwerk simuliert wird, oder dass von der Datenverarbeitungseinrichtung ein neuronales Feedforward-Netzwerk mit einem Faltungsnetzwerk simuliert wird, oder dass von der Datenverarbeitungseinrichtung ein neuronales Faltungsnetzwerk mit einer ersten Faltungsschicht mit nachfolgender Pooling-Schicht und einer nachfolgenden zweiten Faltungsschicht mit mehreren nachfolgenden vollständig verknüpften Schichten simuliert wird.

10. Verfahren zum Bestimmen wenigstens eines geotechnischen Parameters nach einem der Ansprüche 6 - 9, **dadurch gekennzeichnet, dass** vor dem Eingeben des Abschnitts der zeitlichen Folge von Messwerten zum Bestimmen des wenigstens einen geotechnischen Parameters die Gewichte in dem Trainingsverfahren eingestellt werden, indem
a) für eine vorgegebene Auswahl einer Vielzahl von Ausbruchmaterial-Proben unterschiedlicher Zusammensetzungen mit unterschiedlichen geotechnischen Parametern jeweils:
a1) der wenigstens eine geotechnische Parameter der Ausbruchmaterial-Probe gemessen oder bestimmt und als Ziel-Parameterwert gespeichert wird,
a2) der Anprallkörper derart in einem Förderstrom der Ausbruchmaterial-Probe angeordnet wird, dass er zumindest teilweise in das Ausbruchmaterial eintaucht, wobei das Ausbruchmaterial in dem Förderstrom eine vorgegebene Strömungsgeschwindigkeit aufweist, und eine Messgröße, die einer von dem Ausbruchmaterial des Förderstroms der Ausbruchmaterial-Probe auf den Anprallkörper ausgeübten Kraft entspricht, gemessen wird, wobei eine zeitliche Folge von Messwerten der Messgröße erfasst wird,
a3) Paarungen aus jeweils einem Abschnitt der Folge von Messwerten in Zuordnung zu dem Ziel-Parameterwert gespeichert werden,
b) in dem von der Datenverarbeitungseinrichtung implementierten neuronalen Netzwerk den Gewichten Anfangswerte zugewiesen werden,
c) für eine Paarung aus einem Abschnitt der Folge von Messwerten und zugeordnetem Ziel-Parameterwert:
c1) aus dem Abschnitt der Folge von Messwerten der wenigstens eine geotechnische Parameter bestimmt wird, indem der Abschnitt der zeitlichen Folge von Messwerten von der das neuronale Netzwerk implementierenden Datenverarbeitungseinrichtung derart verarbeitet wird, dass wenigstens ein dem wenigstens einen geotechnischen Parameter entsprechender Ausgabewert ausgegeben wird,
c2) dann die Differenz zwischen dem Ausgabewert und dem zugeordneten Ziel-Parameterwert bestimmt wird und
c3) in Abhängigkeit von der Differenz die Gewichte geändert werden,
wobei die Schritte c1) bis c3) für eine nächste Paarung aus einem Abschnitt der Folge von Messwerten und zugeordnetem Ziel-Parameterwert wiederholt werden, wobei die Auswahl der Paarungen in zufälliger Reihenfolge erfolgt,
wobei die Schritte c1) bis c3) für einen vorgegebenen Anteil der im Schritt a) gespeicherten Paarungen ausgeführt werden, der als Trainingsmenge dient.

11. Verfahren zum Bestimmen wenigstens eines geotechnischen Parameters nach einem der Ansprüche 1 - 10, **dadurch gekennzeichnet, dass** als geotechnischer Parameter ein Setzmaß des Ausbruchmaterials bestimmt wird.

12. Verfahren zum Einstellen der Konsistenz eines konditionierten Ausbruchmaterials einer Erddruckschild-Tunnelbohrmaschine, wobei dem Ausbruchmaterial für dessen Konditionierung in der Abbaukammer ein Konditionierungsmittel zugemischt wird,
das konditionierte Ausbruchmaterial mit Fördervorrichtungen aus der Abbaukammer gefördert wird, wobei in einem auf einer der Fördervorrichtungen ausgebildeten offenen Förderstrom wenigstens ein geotechnischer Parameter, vorzugsweise ein Setzmaß, nach einem Verfahren nach einem der Ansprüche 1 - 11 automatisiert bestimmt wird, und
in Abhängigkeit von dem so bestimmten geotechnischen Parameter die Menge des zuzumischenden Konditionierungsmittels geregelt wird.

13. Verfahren zum Klassieren eines Ausbruchmaterials einer Tunnelbohrmaschine, wobei das konditionierte Ausbruchmaterial mit wenigstens einer Fördervorrichtungen aus der Abbaukammer gefördert wird, wobei in einem auf einer der Fördervorrichtungen ausgebildeten offenen Förderstrom wenigstens ein geotechnischer Parameter, vorzugsweise ein Setzmaß, nach einem Verfahren nach einem der Ansprüche 1 - 11 automatisiert bestimmt wird, und das Ausbruchmaterial in Abhängigkeit von dem bestimmten geotechnischen Parameter klassiert wird.

14. Vorrichtung zum Bestimmen wenigstens eines geotechnischen Parameters eines in einer Tunnelbohrmaschine anfallenden Ausbruchmaterials, insbesondere zur Anwendung eines Verfahrens nach einem der Ansprüche 1 bis 11, umfassend:
eine Fördervorrichtung zum Transportieren des Ausbruchmaterials derart, dass in einem offenen Förderkanal (2) ein Förderstrom (3) des Ausbruchmaterials mit einer Strömungsgeschwindigkeit ausgebildet wird, wobei die Fördervorrichtung eine Einrichtung zum Einstellen einer vorgegebenen Strömungsgeschwindigkeit und/oder eine Einrichtung zum Messen der Strömungsgeschwindigkeit aufweist,
ein in dem offenen Förderkanal (2) angeordneter Anprallkörper (5), der zumindest teilweise in den Förderstrom (3) des Ausbruchmaterials eintaucht,
eine mit dem Anprallkörper (5) verbundene Messvorrichtung (7) zum Messen einer Messgröße, die einer von dem Ausbruchmaterial des Förderstroms (3) auf den Anprallkörper (5) ausgeübten Kraft entspricht,
eine mit der Messvorrichtung (7) gekoppelte Vorrichtung zum Erfassen von Abtastwerten der Messgröße, zum Umwandeln der Abtastwerte der Messgröße in digitale Messwerte und zum Ausgeben einer zeitlichen Folge von digitalen Messwerten,
eine Speichervorrichtung zum Zwischenspeichern der Folge von digitalen Messwerten und
eine mit der Speichervorrichtung gekoppelte Datenverarbeitungseinrichtung, die so konfiguriert ist, dass sie einen Abschnitt der Folge von digitalen Messwerten verarbeitet und wenigstens einen dem wenigstens einen geotechnischen Parameter entsprechenden Ausgabewert ausgibt.

15. Vorrichtung zum Bestimmen wenigstens eines geotechnischen Parameters nach Anspruch 14, **dadurch gekennzeichnet, dass** die Datenverarbeitungseinrichtung so konfiguriert ist, dass sie aus dem Abschnitt der Folge von digitalen Messwerten eine Matrix von Eingabedaten erzeugt und verarbeitet und wenigstens einen dem wenigstens einen geotechnischen Parameter entsprechenden Ausgabewert ausgibt und bei der Verarbeitung entweder die eingestellte vorgegebene Strömungsgeschwindigkeit oder ein gemessener Wert der Strömungsgeschwindigkeit berücksichtigt,
wobei die Datenverarbeitungseinrichtung ein trainiertes neuronales Netzwerk implementiert, das mehrere Schichten von Knoten aufweist, wobei sämtlichen Verknüpfungen zwischen den Knoten Gewichte und den Knoten vorgegebene Aktivierungsfunktionen zugeordnet sind, wobei vorab die Gewichte mittels eines Trainingsverfahrens eingestellt worden sind,
wobei die Matrix von Eingabedaten einer Eingabeschicht von Knoten des neuronalen Netzwerks eingegeben und der wenigstens eine dem wenigstens einen geotechnischen Parameter entsprechende Ausgabewert an wenigstens einem Knoten einer Ausgabeschicht des neuronalen Netzwerks ausgegeben wird.

16. Vorrichtung zum Bestimmen wenigstens eines geotechnischen Parameters nach Anspruch 14 oder 15, **dadurch gekennzeichnet, dass** der Anprallkörper (5) als Kugel, vorzugsweise als Stahlkugel mit einem Gewicht von 500 g bis 2000 g, vorzugsweise 800 g bis 1000 g, ausgebildet ist.

17. Vorrichtung zum Bestimmen wenigstens eines geotechnischen Parameters nach Anspruch 16, **dadurch gekennzeichnet, dass** die Kugel (5) an einem schwenkbar aufgehängten Stab oder einem Zugseil (6) befestigt ist und ein Sensor (7) zum Erfassen der Messgröße mit dem Stab beziehungsweise dem Zugseil (6) gekoppelt ist, wobei die Messgröße einer von dem Stab beziehungsweise dem Zugseil (6) aufgenommenen Zugkraft entspricht.

18. Vorrichtung zum Bestimmen wenigstens eines geotechnischen Parameters nach Anspruch 15, **dadurch gekennzeichnet, dass** die Datenverarbeitungseinrichtung so konfiguriert ist, dass sie ein neuronales Feedforward-Netzwerk mit einem eindimensionalen Faltungsnetzwerk simuliert, wobei bevorzugt die Datenverarbeitungseinrichtung so konfiguriert ist, dass sie ein neuronales Faltungsnetzwerk mit einer ersten Faltungsschicht mit nachfolgender Pooling-Schicht und einer nachfolgenden zweiten Faltungsschicht mit mehreren nachfolgenden vollständig verknüpften Schichten simuliert.
